# EUROPEAN PATENT APPLICATION

(11) **EP 1 705 181 A2**
(43) Date of publication of application: **27.09.2006**
(21) Application number: 06005889.8
(22) Date of filing: 22.03.2006
(51) Int. Cl.: C07H 13/04

(54) **Production method of 2-deoxy-L-ribofuranosyl chloride compound**

(30) Priority: 23.03.2005 JP 2005084808
(71) Applicant: Ajinomoto Co., Inc., Tokyo (JP)
(72) Inventor: Nishikawa, Toshihiro Ajinomoto Co., Inc., Kawasaki-shi Kanagawa 210-8681 (JP); Torii, Takayoshi Ajinomoto Co., Inc., Kawasaki-shi Kanagawa 210-8681 (JP); Onishi, Tomoyuki Ajinomoto Co., Inc., Kawasaki-shi Kanagawa 210-8681 (JP)
(74) Representative: Strehl Schübel-Hopf & Partner

(57) **Abstract**

2-Deoxy-L-ribofuranosyl chloride compounds may be produced on an industrial scale by dehalogenating a 2-deoxy-2-halo-L-arabinofuranose compound to give a 2-deoxy-L-ribofuranose compound, and then reacting this compound with a chlorinating agent.

## Description

### FIELD OF THE INVENTION

The present invention relates to method of producing a 2-deoxy-L-ribofuranosyl chloride compound. More particularly, the present invention relates to intermediate compounds which are useful for the production of such a 2-deoxy-L-ribofuranosyl chloride compound and methods of producing such an intermediate compound.

### DISCUSSION OF THE BACKGROUND

A 2-deoxy-L-ribofuranosyl chloride compound (the compound of the formula (3) which is described below) is useful as a synthetic intermediate for various L-nucleoside compounds (*see, e.g.,* Hodge R Anthony Vere, Current Opinion in Investigational Drugs, vol. 5, p. 232 (2004)) known as hepatitis B agents *(see, e.g.,* S. Fujimori et al., Nucleosides & Nucleotides, vol. 11, p. 341-349 (1992) and J. Smejkal et al., Collection of Czechoslovak Chemical Communications, vol. 29, p. 2809 (1964)).

For production of a 2-deoxy-L-ribofuranosyl chloride compound, for example, a three-step production method via 1-alkyl-2-deoxy-L-ribofuranose using 2-deoxy-L-ribose as a starting material is known *(see, e.g.,* S. Fujimori et al., Nucleosides & Nucleotides, vol. 11, p. 341-349 (1992) and M. Hoffer, Chem. Ber., vol. 93, p. 2777 (1960)).

However, since 2-deoxy-L-ribose does not occur naturally, unlike the corresponding D-form, 2-deoxy-L-ribose and derivative compounds thereof need to be produced from L-arabinose, L-ribose, L-ascorbic acid, D-galactose, D-arabinose, 2-deoxy-D-ribose, D-xylose and the like, which are naturally occurring optically active compounds, as starting materials, or produced separately utilizing an asymmetric reaction. Consequently, the above-mentioned methods require quite many steps for the production of the 2-deoxy-L-ribofuranosyl chloride compound, and are not entirely satisfactory as efficient production methods.

As a production method that does not pass through 2-deoxy-L-ribose and 1-alkyl-2-deoxy-L-ribofuranose, for example, a method of directly producing a 2-deoxy-L-ribofuranosyl chloride compound, wherein the 1-position, the 3-position, and the 5-position are protected by an acyl group, with rearrangement of a phenylthioorthoester derivative under radical reduction conditions as a key reaction, which is synthesized in four steps from L-arabinose as a starting material, is known *(see, e.g.,* M. E. Jung et al, Organic Letters, vol. 1, p. 1517 (1999)). According to this method, the object 2-deoxy-L-ribofuranosyl chloride compound can be obtained in six steps from the starting material, L-arabinose. However, this method requires use of ethanethiol and tributyltin hydride, which are difficult to use industrially due to the offensive odor, ignitability, and toxicity, and therefore, the method is not satisfactory from the industrial viewpoints.

Furthermore, a method of producing a 2-deoxy-L-ribofuranosyl chloride compound in eight steps from D-xylose as a starting material with rearrangement (Payne rearrangement) of 5-bromo-2,5-dideoxy-D-threo-pentono-1,4-lactone under basic conditions as a key reaction is known *(see* WO05/21281). However, this method affords a low total yield of 9%, and is not satisfactory from the industrial and economical viewpoints.

In the meantime, it has been reported that a 2-bromo-2-deoxy-1-methyl-D-arabinofuranose compound can be obtained by reacting a 1-methyl-D-arabinofuranose compound derived from D-arabinose with dibromomethyl methyl ether in the presence of zinc bromide *(see* K. Bock et al., Acta Chem. Scand. B, vol. 29, p. 185 (1975)).

It is known that dibromomethylmethylether used for the reaction here can be synthesized by dibrominating methyl formate with 2,2,2-tribromo-2,2-dehydro-1,3,2-dioxaphosphol prepared in two steps from catechol (*see* G. Hans et al., J. Prakt. Chem., vol. 29, p. 315 (1965)). However, the production of dibromomethyl methyl ether requires complicated operations, and the yield is not high. Thus, the production method of the above-mentioned 2-bromo-2-deoxy-1-methyl-D-arabinofuranose compound using it is not entirely industrially satisfactory. In addition, there is no report on the synthesis of the corresponding L-form.

Thus, there remains a need for a method for producing a compound of formula (3) which may be used on an industrial scale. There also remains a need for intermediates useful for such a method and for methods of producing such intermediates.

### SUMMARY OF THE INVENTION

Accordingly, it is one object of the present invention to provide novel methods of producing a 2-deoxy-L-ribofuranosyl chloride compound.

It is another object of the present invention to provide novel methods of producing a 2-deoxy-L-ribofuranosyl chloride compound, which are suitable for industrial production.

It is another object of the present invention to provide novel intermediate compounds which are useful for the production of such a 2-deoxy-L-ribofuranosyl chloride.

It is another object of the present invention to provide novel methods of producing such an intermediate compound.

These and other objects, which will become apparent during the following detailed description, have been achieved by the inventors' discovery that a 2-deoxy-L-ribofuranosyl chloride compound can be produced efficiently by dehalogenating a 2-deoxy-2-halo-L-arabinofuranose compound to give a 2-deoxy-L-ribofuranose compound, and reacting this compound with a chlorinating reagent.

The 2-deoxy-2-halo-L-arabinofuranose compound is a novel substance and is an important intermediate compound useful for the production of a 2-deoxy-L-ribofuranosyl chloride compound.

Furthermore, the present inventors have found that the 2-deoxy-2-halo-L-arabinofuranose compound can be efficiently produced from a known L-arabinofuranose compound or L-arabinofuranosyl halide compound by reacting the compound in the presence of a particular halogenating reagent and a particular acid, and further reacting the resulting compound with an alcohol.

Accordingly, the present invention provides the following:
(1) A method of producing a 2-deoxy-L-ribofuranosyl chloride compound represented by formula (3): 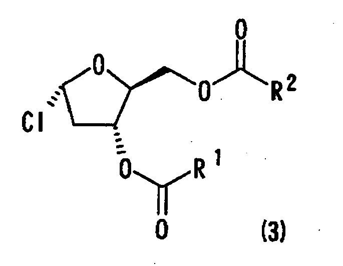 wherein R¹ and R² are each independently an aryl group optionally having one or more substituents, an alkyl group optionally having one or more substituents, or a hydrogen atom,
   said method comprising:
   (a) dehalogenating a 2-deoxy-2-halo-L-arabinofuranose compound represented by formula (1): 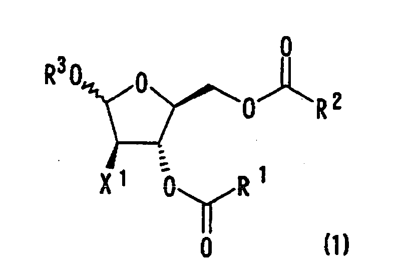 wherein R¹ and R² are as defined above, R³ is an alkyl group or an aralkyl group, and X¹ is a halogen atom, to obtain a 2-deoxy-L-ribofuranose compound represented by formula (2): 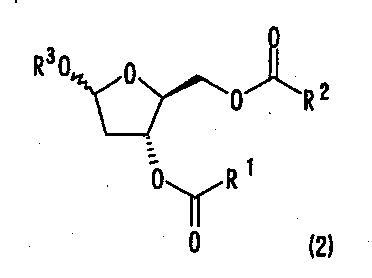 wherein R¹, R², and R³ are as defined above; and
   (ii) reacting said 2-deoxy-L-ribofuranose compound represented by formula (2) with a chlorinating reagent, to obtain said compound of formula (3).
(2) The method of (1) above, wherein the dehalogenating is carried out in the presence of at least one member selected from the group consisting of a phosphite, hypophosphorous acid, a hypophosphite, a nickel catalyst, a palladium catalyst, and mixtures thereof.
(3) The method of (1) above, wherein the chlorinating reagent is at least one member selected from the group consisting of hydrogen chloride, boron chloride, magnesium chloride, aluminum chloride, trialkylsilyl chloride, phosphorus trichloride, phosphorus pentachloride, phosphorus oxychloride, thionyl chloride, sulfuryl chloride, titanium tetrachloride, zinc chloride, acetyl chloride, and mixtures thereof.
(4) The method of (1) above, wherein X¹ is a bromine atom.
(5) The method of (1) above, wherein both R¹ and R² are an aryl group optionally having one or more substituents.
(6) A method of producing a 2-deoxy-2-halo-L-arabinofuranose compound represented by formula (1): 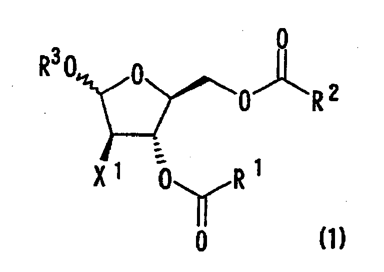 wherein R¹ and R² are each independently an aryl group optionally having one or more substituents, an alkyl group optionally having one or more substituents, or a hydrogen atom, R³ is an alkyl group or an aralkyl group, and X¹ is a halogen atom,
   said method comprising:
   (i) reacting:
      (a) an L-arabinofuranose compound represented by formula (4): 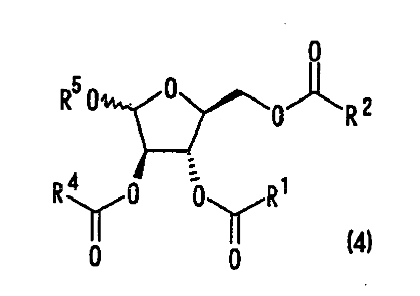 wherein R¹ and R² are as defined above, R⁴ is an aryl group optionally having one or more substituents, an alkyl group optionally having one or more substituents, or a hydrogen atom, and R⁵ is an alkyl group or an aralkyl group, or
      (b) an L-arabinofuranosyl halide compound represented by formula (5): 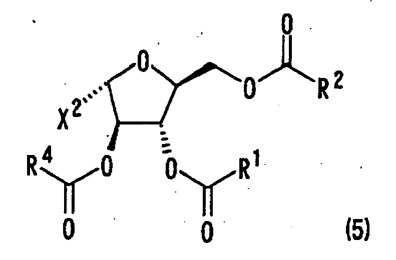 wherein R¹, R², and R⁴ are as defined above, and X² is a halogen atom,
         with a halogenating reagent and an acid, to obtain a compound represented by formula (6): 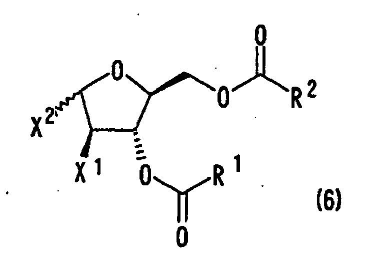 wherein X², R¹, and R² are as defined above, and X¹ is a halogen atom; and
   (ii) reacting the compound represented by formula (6) with an alcohol, to obtain said compound of formula (1).
(7) The method of (6) above, wherein the halogenating reagent is at least one member selected from the group consisting of hydrogen bromide, phosphorus tribromide, acetyl bromide, and mixtures thereof.
(8) The method of (6) above, wherein the acid is at least one member selected from the group consisting of zinc chloride, zinc bromide, aluminum bromide, and mixtures thereof.
(9) A method of producing a 2-deoxy-L-ribofuranose compound represented by formula (2): 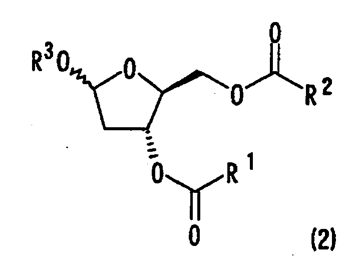 wherein R¹ and R² are each independently an aryl group optionally having one or more substituents, an alkyl group optionally having one or more substituents, or a hydrogen atom, and R³ is an alkyl group or an aralkyl group,
   said method comprising:
   (i) dehalogenating a 2-deoxy-2-halo-L-arabinofuranose compound represented by formula (1): 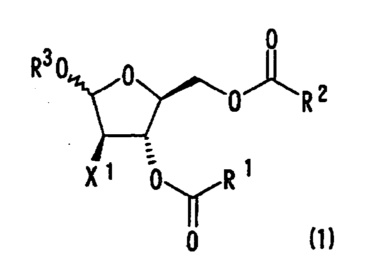 wherein R¹, R², and R³ are as defined above, and X¹ is a halogen atom.
(10) The method of (9) above, wherein the dehalogenating is carried out in the presence of at least one member selected from a phosphite, hypophosphorous acid, a hypophosphite, a nickel catalyst, a palladium catalyst, and mixtures thereof.
(11) The method of (9) above, wherein X¹ is a bromine atom.
(12) The method of (9) above, wherein both R¹ and R² are an aryl group optionally having one or more substituents.
(13) A method of producing a 2-deoxy-2-halo-L-arabinofuranose compound represented by formula (1'): 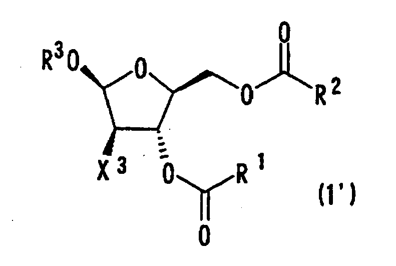 wherein R¹ and R² are each independently an aryl group optionally having one or more substituents, an alkyl group optionally having one or more substituents, or a hydrogen atom, R³ is an alkyl group or an aralkyl group and X³ is a bromine atom,
   said method comprising:
   (i) reacting
      (a) an L-arabinofuranose compound represented by formula (4): 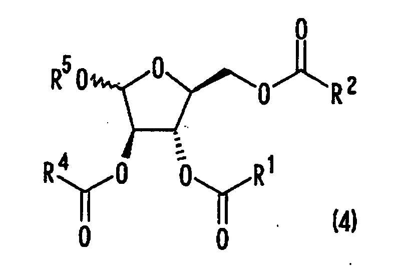 wherein R¹ and R² are as defined above, R⁴ is an aryl group optionally having one or more substituents, an alkyl group optionally having one or more substituents, or a hydrogen atom, and R⁵ is an alkyl group or an aralkyl group, or
      (b) an L-arabinofuranosyl halide compound represented by formula (5): 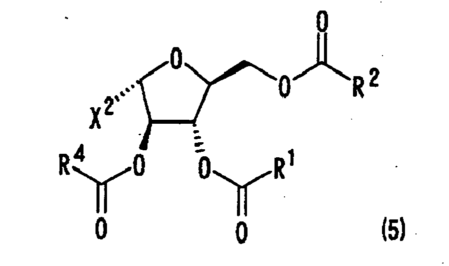 wherein R¹, R², and R⁴ are as defined above, and X² is a halogen atom,
      with an acid and at least one halogenating reagent selected from the group consisting of hydrogen bromide, phosphorus tribromide, acetyl bromide, and mixtures thereof, to obtain a compound represented by formula (6): 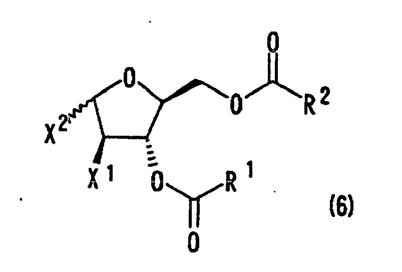 wherein X², R¹, and R² are as defined above, and X¹ is a halogen atom; and
   (ii) reacting said compound represented by formula (6) with an alcohol, to obtain said compound of formula (1').
(14) The method of (13) above, wherein the acid is at least one member selected from the group consisting of zinc chloride, zinc bromide, aluminum bromide, and mixtures thereof.
(15) The method of (13) above, wherein X² is a bromine atom.
(16) A 2-deoxy-2-halo-L-arabinofuranosyl halide compound represented by formula (6): 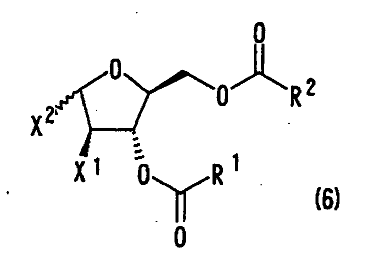 wherein R¹ and R² are each independently an aryl group optionally having one or more substituents, an alkyl group optionally having one or more substituents, or a hydrogen atom, and X¹ and X² are each independently a halogen atom.
(17) The compound of (16) above, wherein both X¹ and X² are a bromine atom.
(18) The compound of (16) above, wherein both R¹ and R² are an aryl group optionally having one or more substituents.
(19) A 2-deoxy-2-halo-L-arabinofuranose compound represented by formula (1): 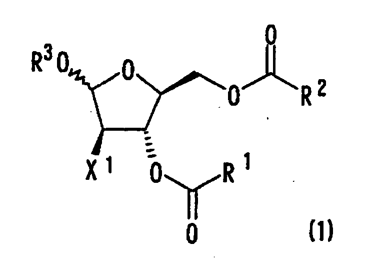 wherein R¹ and R² are each independently an aryl group optionally having one or more substituents, an alkyl group optionally having one or more substituents, or a hydrogen atom, R³ is an alkyl group or an aralkyl group, and X¹ is a halogen atom.
(20) The compound of (19) above, wherein X¹ is a bromine atom.
(21) The compound of (19) above, wherein both R¹ and R² are an aryl group optionally having one or more substituents.
(22) A method of producing a compound represented by formula (7): 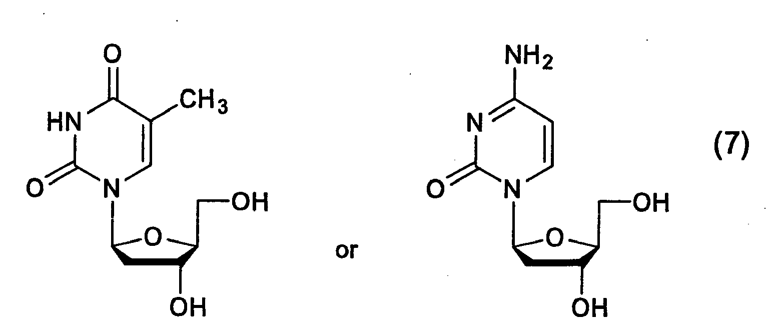 by converting a compound represented by formula (3): 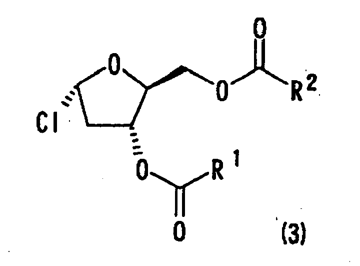 wherein R¹ and R² are each independently an aryl group optionally having one or more substituents, an alkyl group optionally having one or more substituents, or a hydrogen atom,
   into the compound represented by formula (7), wherein the compound represented by formula (3) is prepared by a method comprising:
   (a) dehalogenating a 2-deoxy-2-halo-L-arabinofuranose compound represented by formula (1): 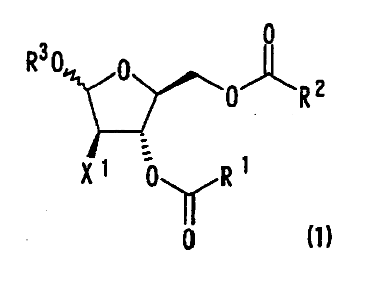 wherein R¹ and R² are as defined above, R³ is an alkyl group or an aralkyl group, and X¹ is a halogen atom, to obtain a 2-deoxy-L-ribofuranose compound represented by formula (2): 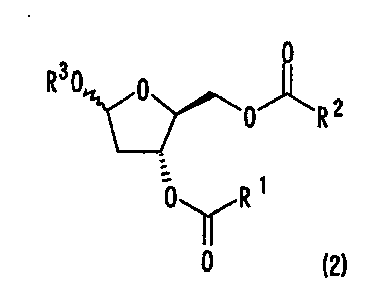 wherein R¹, R², and R³ are as defined above; and
   (ii) reacting said 2-deoxy-L-ribofuranose compound represented by formula (2) with a chlorinating reagent, to obtain said compound of formula (3).

According to the present invention, an efficient method of producing a 2-deoxy-L-ribofuranosyl chloride compound, which is suitable for industrial production, and further, an intermediate compound important for the production of a 2-deoxy-L-ribofuranosyl chloride compound and a production method of the intermediate compound are provided.

### DETAILED DESCRIPTION OF THE PREFERRED EMBODIMENTS

In the formulas in the present invention, R³ and R⁵ are each independently an alkyl group or an aralkyl group. As the alkyl group, a straight chain or branched chain saturated alkyl group having 1 to 8 carbon atoms can be mentioned. As the aralkyl group, an aralkyl group having 7 to 15 carbon atoms optionally having one or more substituents can be mentioned.

As R³ and R⁵, a straight chain or branched chain saturated alkyl group having 1 to 4 carbon atoms (*e.g.*, methyl group, ethyl group, propyl group, isopropyl group, butyl group, or 1-methyl-1-propyl group) or a benzyl group is particularly preferable.

In the formulas in the present invention, R¹, R², and R⁴ are each independently an aryl group optionally having one or more substituents, an alkyl group optionally having one or more substituents or a hydrogen atom.

As the alkyl group of the alkyl group optionally having one or more substituents, a straight chain or branched chain saturated alkyl group having 1 to 8 carbon atoms can be mentioned.

As the aryl group of the aryl group optionally having one or more substituents, an aryl group having 6 to 14 carbon atoms can be mentioned.

The substituent for the above-mentioned alkyl group or aryl group having one or more substituents is not particularly limited and, for example, an alkoxy group (preferably having 1 to 4 carbon atoms), a nitro group, an alkyl group (preferably having 1 to 4 carbon atoms), a halogen atom, and the like can be mentioned.

As the aryl group optionally having one or more substituents, a phenyl group optionally having one or more substituents is preferable, and a phenyl group, a p-methylphenyl group, and a p-chlorophenyl group are particularly preferable.

As the alkyl group optionally having one or more substituents, a substituted or unsubstituted straight chain or branched chain saturated alkyl group having 1 to 4 carbon atoms, namely, a methyl group, an ethyl group, a propyl group, and a butyl group are preferable.

In the formulas in the present invention, X¹ is a halogen atom. As the halogen atom, a fluorine atom, a chlorine atom, a bromine atom, and an iodine atom can be mentioned. Preferred are a bromine atom and an iodine atom, and particularly preferred is a bromine atom.

In the formulas in the present invention, X² is a halogen atom. As the halogen atom, a fluorine atom, a chlorine atom, a bromine atom, and an iodine atom can be mentioned. Preferred are a fluorine atom, a chlorine atom, and a bromine atom, and particularly preferred is a bromine atom.

The L-arabinofuranose compound represented by the formula (4) and the L-arabinofuranosyl halide compound represented by the formula (5), which are used as starting materials in the present invention, can be easily produced from L-arabinose by two or three steps according to a known method *(e.g., see* WO98/39347).

A method for producing a 2-deoxy-2-halo-L-arabinofuranose compound represented by the formula (1) by reacting an L-arabinofuranose compound represented by the formula (4) or L-arabinofuranosyl halide compound represented by the formula (5) in the presence of a particular halogenating reagent and a particular acid, and then further reacting the resulting compound with alcohol is explained in the following. This step can be expressed by the following scheme. 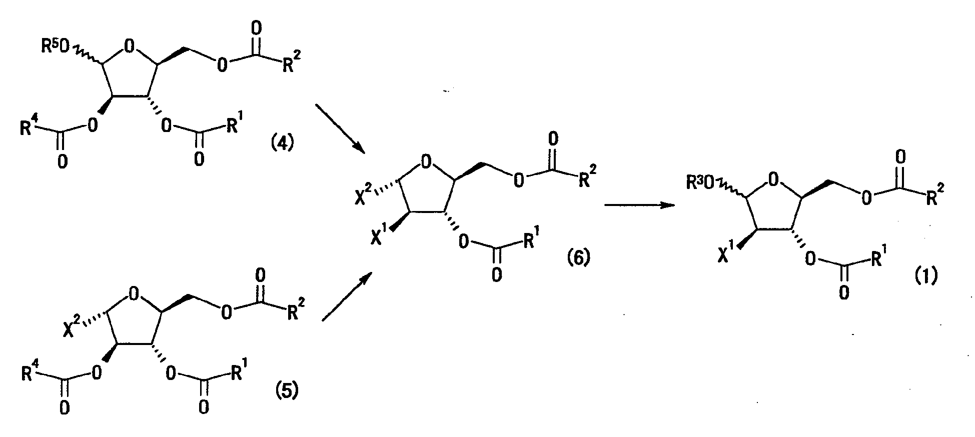 wherein each symbol is as defined above.

The compound represented by the formula (6) is an intermediate produced after reaction in the presence of a particular halogenating reagent and a particular acid in the above-mentioned reaction step. It may be once isolated in this step before use, but generally, the isolation is not necessary and the compound can be converted to a compound represented by the formula (1) by reaction with an alcohol in the reaction solution. It is needless to say that the case where a compound represented by the formula (6) is isolated from the reaction system in this step is also encompassed in the present invention.

As the halogenating reagent to be used in the present invention when X¹ is a fluorine atom, fluorine, hydrogen fluoride, sulfur tetrafluoride, and dialkylaminosulfur trifluoride can be mentioned.

When X¹ is a chlorine atom, the halogenating reagent includes, for example, chlorine, hydrogen chloride, lithium chloride, boron chloride, ammonium chloride, magnesium chloride, aluminum chloride, trialkylsilyl chloride, phosphorus trichloride, phosphorus pentachloride, phosphorus oxychloride, thionyl chloride, sulfuryl chloride, calcium chloride, titanium tetrachloride, zinc chloride, acetyl chloride, acetoxyisobutyryl chloride, dichloromethyl butyl ether, dichloromethyl methyl ether, 2-chloro-2,2-dehydro-1,3,2-dioxaphosphol, and 2,2,2-trichloro-2,2-dehydro-1,3,2-dioxaphosphol.

When X¹ is a bromine atom, the halogenating reagent includes, for example, bromine, hydrogen bromide, lithium bromide, boron bromide, ammonium bromide, magnesium bromide, aluminum bromide, trialkylsilyl bromide, phosphorus tribromide, phosphorus pentabromide, phosphorus oxybromide, thionyl bromide, calcium bromide, titanium tetrabromide, zinc bromide, acetyl bromide, acetoxyisobutyryl bromide, dibromomethyl butyl ether, dibromomethyl methyl ether, 2-bromo-2,2-dehydro-1,3,2-dioxaphosphol, and 2,2,2-tribromo-2,2-dehydro-1,3,2-dioxaphosphol. Particularly, hydrogen bromide, boron bromide, trialkylsilyl bromide, phosphorus tribromide, phosphorus pentabromide, phosphorus oxybromide, thionyl bromide, acetyl bromide, acetoxyisobutyryl bromide, dibromomethyl butyl ether, and dibromomethyl methyl ether are preferable, and hydrogen bromide, phosphorus tribromide, and acetyl bromide are most preferable.

When X¹ is an iodine atom, iodine, hydrogen iodide, lithium iodide, ammonium iodide, magnesium iodide, aluminum iodide, trialkylsilyl iodide, phosphorus triiodide, calcium iodide, titanium tetraiodide, zinc iodide, and 2-chloro-2,2-diiodo-2,2-dehydro-1,3,2-dioxaphosphol can be mentioned. Particularly, hydrogen iodide, trialkylsilyl iodide, and phosphorus triiodide are preferable.

When a 2-deoxy-L-ribofuranose compound or a 2-deoxy-L-ribofuranosyl chloride compound is to be produced in the present invention, dehalogenation should be carried out in the next step. Therefore, as X¹ in the present invention, a bromine atom or an iodine atom is preferable in view of ease of dehalogenation. For the synthesis of a 2-deoxy-2-halo-L-arabinofuranose compound, since ease of reaction is markedly influenced by the nucleophilicity of a halogen atom, X¹ in the present invention is preferably a bromine atom or an iodine atom, which are halogen atoms having high nucleophilicity. Taking an economic aspect into consideration, furthermore, a bromine atom is particularly most preferable. As the halogenating reagent in the present invention, at least one member selected from the group consisting of hydrogen bromide, phosphorus tribromide, and acetyl bromide, which are particularly superior from the above-mentioned viewpoint, is most preferably used.

As the acid to be used together with the halogenating reagent in the present invention, for example, hydrogen fluoride, hydrogen chloride, hydrogen bromide, hydrogen iodide, sulfuric acid, methanesulfonic acid, benzenesulfonic acid, p-toluenesulfonic acid, trifluoromethanesulfonic acid, boron trifluoride ether complex, boron trichloride, boron tribromide, magnesium chloride, magnesium bromide, magnesium iodide, aluminum chloride, aluminum bromide, aluminum iodide, trialkylsilyl chloride, trialkylsilyl bromide, trialkylsilyl iodide, titanium tetrachloride, titanium tetrabromide, titanium tetraiodide, zinc chloride, zinc bromide, zinc iodide, and the like can be mentioned. As the acid, at least one acid selected from the group consisting of zinc chloride, zinc bromide, and aluminum bromide is particularly preferable.

When the acid also functions as a halogenating reagent (*e.g.*, when hydrogen bromide is used), other acid may or may not be present.

While the amount of the halogenating reagent or acid to be used is not particularly limited, it is 0.2 to 10 equivalents, preferably 0.5 to 3 equivalents, both in molar ratios, relative to the L-arabinofuranose compound or L-arabinofuranosyl halide compound.

As the reaction solvent, dichloromethane, chloroform, 1,2-dichloroethane, acetonitrile, acetone, ethyl acetate, isopropyl acetate, tetrahydrofuran, diethyl ether, toluene, hexane, heptane, and the like are used alone or as a mixture, with particular preference given to dichloromethane and chloroform. These reaction solvents may contain a protonic solvent (*e.g.*, water, methanol, ethanol, *etc.)* in an amount free from an adverse effect to the reaction. While the reaction temperature varies depending on the combination of the halogenating reagent, acid, and solvent to be used and is not particularly limited, it is preferably 0 to 100°C. The reaction time is not particularly limited, either, and preferred is about 10 minutes to 50 hours. After the completion of the reaction, the reaction is quenched by adding diluted hydrochloric acid, aqueous sodium hydrogen carbonate solution, water, and the like to the reaction mixture as necessary, and the reaction mixture is subjected to work-up such as extraction with a suitable organic solvent and the like.

As mentioned above, a 2-deoxy-2-halo-L-arabinofuranosyl halide compound (6) obtained at this stage can be isolated as a solid from a reaction solution by a method known to those of ordinary skill in the art, such as column chromatography, crystal precipitation, and the like. To increase the yield, however, isolation is generally not necessary, and the reaction solution is preferably used directly in the next step for the reaction with alcohol or, where necessary, used after controlling the amount of a solvent, substituting the solvent and the like.

A method of producing a 2-deoxy-2-halo-L-arabinofuranose compound represented by the formula (1) by reacting the obtained 2-deoxy-2-halo-L-arabinofuranosyl halide compound represented by the formula (6) with an alcohol is now explained.

The glycosyl halide moiety of a 2-deoxy-2-halo-L-arabinofuranosyl halide compound represented by the formula (6) can be easily alcoholyzed by reacting the compound with an alcohol in the presence of an acid. As the alcohol to be used, for example, lower alcohols such as methanol, ethanol, 1-propanol, 1-butanol, 2-propanol, 2-butanol, and the like can be mentioned. The acid to be used for the alcoholysis is not particularly limited and, for example, hydrofluoric acid, hydrochloric acid, hydrobromic acid, hydroiodic acid, sulfuric acid, phosphoric acid, methanesulfonic acid, benzenesulfonic acid, p-toluenesulfonic acid, trifluoromethanesulfonic acid, and the like can be mentioned.

While the reaction temperature is not particularly limited, it is preferably -10°C to 50°C. While the reaction time varies depending on the alcohol to be used and the reaction temperature, when, for example, the reaction is carried out using methanol at room temperature, the reaction completes in about 10 minutes to 2 hours.

The method of isolating an object product from a reaction solution is not particularly limited, and a method those of ordinary skill in the art generally employ can be used. For example, the obtained reaction solution is concentrated where necessary, and the concentrate is extracted with a solvent such as dichloromethane, ethyl acetate, isopropyl acetate, toluene, diethyl ether, tert-butyl methyl ether, pentane, hexane, heptane, and the like or a mixed solvent thereof. The extract is concentrated where necessary, heated to about 40°C to 80°C, and cooled to -20°C to room temperature to allow cooling crystal precipitation to give a 2-deoxy-2-halo-L-arabinofuranose compound as crystals. The compound can also be obtained as a solid by chromatography and the like.

When the 2-deoxy-2-halo-L-arabinofuranose compound is to be further led to a further compound, the reaction solution may be used directly in the next step or, where necessary, used after controlling the amount of a solvent, substituting the solvent and the like, without isolation and purification.

A method of producing a 2-deoxy-L-ribofuranose compound represented by the formula (2) by dehalogenating a 2-deoxy-2-halo-L-arabinofuranose compound represented by the formula (1) is now explained.

The 2-deoxy-2-halo-L-arabinofuranose compound represented by the formula (1) can be dehalogenated by radical reduction using at least one of tributyltin hydride, triethylsilane hydride, a phosphite, hypophosphorous acid, and a hypophosphite, or hydrogenation using a palladium catalyst and the like and/or with a nickel catalyst. As hypophosphite, for example, N-ethylpiperidinium hypophosphite, triethylammonium hypophosphite, hexadecyltrimethylammonium hypophosphite and the like can be mentioned.

When tributyltin hydride or triethylsilane hydride is used as a dehalogenating agent, a radical initiator such as 2,2'-azobisisobutyronitrile, 1,1'-azobis(cyclohexanecarbonitrile), triethylborane, and the like is co-used. The amount of the dehalogenating agent to be used is not particularly limited, and the agent may be reacted in an amount of 1 to 10 molar equivalents relative to the 2-deoxy-2-halo-L-arabinofuranose compound. The amount of the radical initiator to be used is also not particularly limited, and it may be reacted in an amount of 0.05 to 10 molar equivalents relative to the 2-deoxy-2-halo-L-arabinofuranose compound.

As the reaction solvent, methanol, ethanol, 1-propanol, 2-propanol, 1-butanol, 2-butanol, tert-butanol, acetonitrile, acetone, ethylmethylketone, isobutylmethylketone, ethyl acetate, isopropyl acetate, 1,4-dioxane, tetrahydrofuran, diethyl ether, tert-butyl methyl ether, benzene, toluene, hexane, heptane, and the like are used alone or in a mixture, with particular preference given to toluene. The reaction temperature varies depending on the solvent to be used and is not particularly limited. Preferred is a reaction temperature of 0°C to 140°C. While the reaction time varies depending on the solvent to be used and reaction temperature, when, for example, the reaction is carried out using toluene at 110°C, the reaction is completed in about 1 hour to 24 hours.

When one of phosphite, hypophosphorous acid, and hypophosphite is used as a dehalogenating agent, a radical initiator such as 2,2'-azobisisobutyronitrile, 1,1'-azobis(cyclohexanecarbonitrile), 4,4'-azobis(4-cyanovaleric acid), V-50, triethylborane, and the like is co-used. Where necessary, a base such as triethylamine and the like may be co-used. The amount of the dehalogenating agent to be used is not particularly limited, and 1 to 10 molar equivalents thereof may be used relative to the 2-deoxy-2-halo-L-arabinofuranose compound. The amount of the radical initiator to be used is not particularly limited, and 0.05 to 10 molar equivalents, preferably 0.1 to 1.5 molar equivalents, thereof may be used relative to the 2-deoxy-2-halo-L-arabinofuranose compound.

As the reaction solvent, water, methanol, ethanol, 1-propanol, 2-propanol, 1-butanol, 2-butanol, tert-butanol, acetonitrile, acetone, ethylmethylketone, isobutylmethylketone, ethyl acetate, isopropyl acetate, 1,4-dioxane, tetrahydrofuran, diethyl ether, tert-butyl methyl ether, benzene, toluene, hexane, heptane, and the like are used alone or in a mixture, with particular preference given to a mixed solvent of acetonitrile and water. The reaction temperature varies depending on the solvent to be used and is not particularly limited. Preferred is a reaction temperature of 0°C to 140°C. While the reaction time varies depending on the solvent to be used and reaction temperature, when, for example, the reaction is carried out using a mixed solvent of acetonitrile and water at 80°C, the reaction completes in about 1 hour to 24 hours.

When a nickel catalyst is used as a dehalogenating agent, the amount of the nickel catalyst to be used is not particularly limited, and 3 to 20-fold by weight thereof only needs to be added relative to the 2-deoxy-2-halo-L-arabinofuranose compound. Where necessary, a base such as triethylamine and the like may be co-used. As the reaction solvent, water, methanol, ethanol, and the like are used alone or in a mixture. The reaction temperature varies depending on the solvent to be used and is not particularly limited. Preferred is a reaction temperature of 20°C to 100°C. While the reaction time varies depending on the solvent to be used and reaction temperature, when, for example, the reaction is carried out using ethanol at 70°C, the reaction completes in about 1 hour to 24 hours.

When a palladium catalyst is used as a dehalogenating agent, the reaction is carried out under hydrogen atmosphere. The amount of the palladium catalyst to be used is not particularly limited, and 1 to 100 wt% thereof only need to be added relative to the 2-deoxy-2-halo-L-arabinofuranose compound. Where necessary, a base such as triethylamine and the like may be co-used. As the reaction solvent, water, methanol, ethanol, 2-propanol, and the like are used alone or in a mixture. The reaction temperature varies depending on the solvent to be used and is not particularly limited. Preferred is 10°C to 100°C. While the reaction time varies depending on the solvent to be used and reaction temperature, when, for example, the reaction is carried out using 2-propanol at 80°C, the reaction completes in about 1 hour to 24 hours.

In view of safety in the industrial use, at least one of phosphite, hypophosphorous acid, hypophosphite, nickel catalyst, and palladium catalyst is preferably used as a dehalogenating agent.

The method of isolating an object product from a reaction solution is not particularly limited, and a method those of ordinary skill in the art generally employ can be used. For example, the obtained reaction solution is concentrated where necessary, and the concentrate is extracted with a solvent such as dichloromethane, ethyl acetate, isopropyl acetate, toluene, diethyl ether, tert-butyl methyl ether, pentane, hexane, heptane, and the like or a mixed solvent thereof. The extract is concentrated where necessary, heated to about 25°C to 100°C, and cooled to -20°C to room temperature to allow cooling-precipitation to give a 2-deoxy-L-ribofuranose compound as crystals. The compound can also be obtained as a solid by chromatography and the like. In addition, the 2-deoxy-L-ribofuranose compound can also be obtained as crystals by concentrating a reaction solution, where necessary, directly subjecting, without extraction, the concentrate to cooling-precipitation under the above-mentioned conditions, and washing the obtained crystals with a suitable solvent. When the 2-deoxy-L-ribofuranose compound is to be further led to a further compound, the reaction solution may be directly used in the next step or, where necessary, used after controlling the amount of a solvent, substituting the solvent and the like, without isolation and purification.

The 2-deoxy-L-ribofuranose compound represented by the formula (2) is a known compound useful as a synthetic intermediate for, for example, various L-nucleoside compounds known as hepatitis B agents *(see, e.g.,* S. Fujimori et al., Nucleosides & Nucleotides, vol. 11, p. 341-349 (1992)), and is known to be converted to an intermediate in an advanced form by, for example, the following known methods *(see* S. Fujimori et al., Nucleosides & Nucleotides, vol. 11, p. 341-349 (1992), M. Hoffer, Chem. Ber., vol. 93, 2777 (1960), and the like). That is, a 2-deoxy-L-ribofuranosyl chloride compound represented by the formula (3) can be produced by reacting a 2-deoxy-L-ribofuranose compound represented by the formula (2) with a chlorinating reagent.

As the chlorinating reagent, hydrogen chloride, boron chloride, magnesium chloride, aluminum chloride, trialkylsilyl chloride, phosphorus trichloride, phosphorus pentachloride, phosphorus oxychloride, thionyl chloride, sulfuryl chloride, titanium tetrachloride, zinc chloride, acetyl chloride, and the like can be mentioned, with particular preference given to hydrogen chloride and acetyl chloride. They may be used in a combination of two more kinds thereof. As the reaction solvent, acetic acid, 1,4-dioxane, tetrahydrofuran, ethyl acetate, dichloromethane, and the like are used alone or in a mixture, with particular preference given to acetic acid and ethyl acetate. While the amount of the chlorinating reagent to be used varies depending on the chlorinating reagent to be used and combination of solvents, it is 1 to 10 molar equivalents, preferably 1 to 5 molar equivalents. The reaction time is not particularly limited, but it is preferably about 10 minutes to 50 hours.

The method of isolating an object product from a reaction solution after completion of the reaction is not particularly limited, and a method those of ordinary skill in the art generally employ can be used. For example, the obtained reaction solution is concentrated where necessary, and the concentrate is extracted with a solvent such as dichloromethane, ethyl acetate, isopropyl acetate, toluene, diethyl ether, tert-butyl methyl ether, pentane, hexane, heptane, and the like or a mixed solvent thereof. Where necessary, the extract is concentrated, heated to about 25°C to 100°C, and cooled to -20°C to room temperature to allow cooling crystal precipitation to give a 2-deoxy-L-ribofuranosyl chloride compound as crystals. The compound can also be obtained as a solid by chromatography and the like. In addition, the 2-deoxy-L-ribofuranosyl chloride compound can also be obtained as crystals by concentrating a reaction solution, where necessary, directly subjecting, without extraction, the concentrate to cooling-precipitation under the above-mentioned conditions, and washing the obtained crystals with a suitable solvent.

As noted above, the 2-deoxy-L-ribofuranosyl chloride compounds represented by formula (3) are useful as a synthetic intermediates for various hepatitis B agents *(see, e.g.*, S. Fujimori et al., Nucleosides & Nucleotides, vol. 11, p. 341-349 (1992) and J. Smejkal et al., Collection of Czechoslovak Chemical Communications, vol. 29, p. 2809 (1964)). Accordingly, in another embodiment, the present invention provides methods for preparing a compound represented by formula (7): 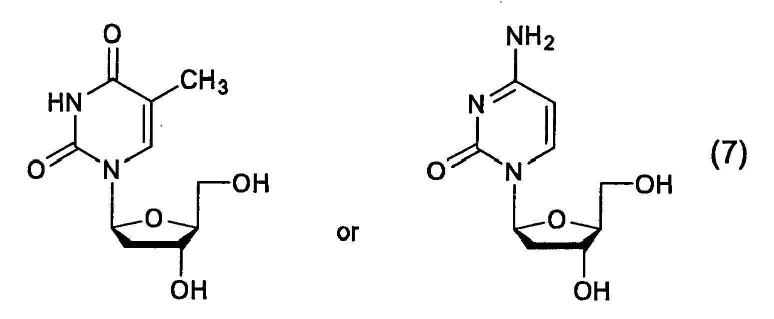 by converting a compound represented by formula (3) into a compound represented by formula (7). Methods for the conversion of the compound represented by formula (3) into a compound represented by formula (7) are described in, *e.g.,* S. Fujimori et al., Nucleosides & Nucleotides, vol. 11, p. 341-349 (1992); and J. Smejkal et al., Collection of Czechoslovak Chemical Communications, vol. 29, p. 2809 (1964), which are incorporated herein by reference in their entireties.

Other features of the invention will become apparent in the course of the following descriptions of exemplary embodiments which are given for illustration of the invention and are not intended to be limiting thereof.

### EXAMPLES

### Example 1. Synthesis of 2-deoxy-2-bromo-3,5-di-O-toluoyl-α-L-arabinofuranosyl bromide

To a solution of methyl-2,3,5-tri-O-toluoyl-L-arabinofuranoside (240 mg, 0.46 mmol), which was synthesized according to the method described in WO98/39347, in chloroform (4.6 mL) were added zinc bromide (416 mg,1.84 mmol) and phosphorus tribromide (0.18 mL, 1.84 mmol), and the mixture was stirred under heating at 45 °C for 19 hours. After the completion of the reaction, the reaction mixture was cooled to room temperature and separated by adding dichloromethane (5 mL) and 2 M aqueous hydrochloric acid solution (25 mL). The aqueous layer was extracted again with dichloromethane (5 mL). The organic layers were combined and washed with saturated aqueous sodium hydrogencarbonate solution (25 mL). The aqueous layer was extracted again with dichloromethane (5 mL). The organic layers were combined, dried over anhydrous sodium sulfate, and concentrated under reduced pressure to give an oily substance (218 mg). The amount of the object compound in this oily substance was determined to be 113 mg (yield 48%) by quantitation using an NMR internal standard. In addition, a small amount of sample was purified by chromatography (elution 30:1 hexane-ethyl acetate).
¹H-NMR (CDCl₃) δ 2.40 (s, 3H), 2.43 (s, 3H), 2.46 (s, 3H), 4.74 (dd, J= 12.2,5.6 Hz, 1H), 4.85 (dd, J=12.2, 3.5 Hz, 1H), 4.90 (s, 1H), 5.68 (d, J= 3.9 Hz, 1H), 6.73 (s, 1H), 7.22 (d, J= 8.0 Hz, 2H), 7.27 (d, J= 8.0 Hz, 2H), 7.31 (d, J= 8.0 Hz, 2H), 7.99 (d, J= 8.2 Hz, 2H), 8.00 (d, J= 8.2 Hz, 2H), 8.03 (d, J= 8.2 Hz, 2H).

### Example 2. Synthesis of 2-deoxy-2-bromo-3,5-di-O-toluoyl-α-L-arabinofuranosyl bromide

To a solution of bromo-2,3,5-tri-O-toluoyl-α-L-arabinofuranoside (1.0 g, 1.76 mmol), which was synthesized according to the method described in WO98/39347, in chloroform (17.6 mL) were added zinc bromide (794 mg, 3.52 mmol) and phosphorus tribromide (0.34 mL, 3.52 mmol), and the mixture was stirred under heating at 45°C for 22 hours. After the completion of the reaction, the reaction mixture was cooled to room temperature and separated by adding dichloromethane (15 mL) and 2 M aqueous hydrochloric acid solution (25 mL). The aqueous layer was extracted again with dichloromethane (15 mL). The organic layers were combined and washed with saturated aqueous sodium hydrogencarbonate solution (25 mL). The aqueous layer was extracted again with dichloromethane (15 mL). The organic layers were combined, dried over anhydrous sodium sulfate, and concentrated under reduced pressure to give an oily substance (1.03 g). The amount of the object compound in this oily substance was determined to be 550 mg (yield 61 %) by quantitation using an NMR internal standard.

### Example 3. Synthesis of methyl-2-deoxy-2-bromo-3,5-di-O-toluoyl-β-L-arabinofuranoside and methyl-2-deoxy-2-bromo-3,5-di-O-toluoyl-α-L-arabinofuranoside

To a solution of methyl-2,3,5-tri-O-toluoyl-L-arabinofuranoside (259 mg, 0.5 mmol) in chloroform (5 mL) were added zinc bromide (450 mg, 2.0 mmol) and phosphorus tribromide (0.19 mL, 2.0 mmol), and the mixture was stirred under heating at 45°C for 18 hours. After the completion of the reaction, the reaction mixture was cooled to room temperature. Methanol (5 mL) was added to the solution, and the mixture was stirred for 1 hour. The resulting solution was concentrated, and separated by adding dichloromethane (20 mL) and water (4 mL). The organic layer was washed with water (4 mL) and saturated brine (2 mL), dried over anhydrous magnesium sulfate, and concentrated under reduced pressure. The resulting oily substance was purified by chromatography (elution 20:1→10:1 hexane-ethyl acetate) to give methyl-2-deoxy-2-bromo-3,5-di-O-toluoyl-β-L-arabinofuranoside (140.3 mg, yield 61 %) as white crystals and methyl-2-deoxy-2-bromo-3,5-di-O-toluoyl-α-L-arabinofuranoside (30.0 mg, yield 13%) as a colorless oily substance.
Methyl-2-deoxy-2-bromo-3,5-di-O-toluoyl-β-L-arabinofuranoside
¹H-NMR (CDCl₃) δ 2.39 (s, 3H), 2.43 (s, 3H), 3.46 (s, 3H), 4.33―4.40 (m, 1H), 4.43 (dd, J= 8.2, 4.3 Hz, 1H), 4.54 (dd, J= 11.8, 6.8 Hz, 1H), 4.71 (dd, J=11.8, 4.0 Hz, 1H), 5.02 (d, J= 4.3 Hz, 1H), 5.79 (dd, J= 8.3, 5.5 Hz, 1H), 7.19 (d, J= 7.9 Hz, 2H), 7.25 (d, J= 7.9 Hz, 2H) 7.93 (d, J= 8.2 Hz, 2H), 7.94 (d, J= 8.2 Hz, 2H).
Methyl-2-deoxy-2-bromo-3,5-di-O-toluoyl-α-L-arabinofuranoside
¹H-NMR (CDCl₃) δ 2.40 (s, 3H), 2.42 (s, 3H), 3.45 (s, 3H), 4.24 (d, J= 1.1 Hz, 1H), 4.51―4.56 (m, 1H), 4.63 (dd, J= 11.9, 5.1 Hz, 1H), 4.74 (dd, J= 11.9, 3.8 Hz, 1H), 5.27 (s, 1H), 5.60 (dd, J= 4.7, 1.8 Hz, 1H), 7.21 (d, J= 8.0 Hz, 2H), 7.24 (d, J= 8.0 Hz, 2H) 7.92 (d, J= 8.2 Hz, 2H), 7.99 (d, J= 8.2 Hz, 2H).

### Example 4. Synthesis of methyl-2-deoxy-2-bromo-3,5-di-O-toluoyl-β-L-arabinofuranoside

To a solution of methyl-2,3,5-tri-O-toluoyl-L-arabinofuranoside (259 mg, 0.5 mmol) in chloroform (0.85 mL) was added zinc bromide (225 mg, 1.0 mmol) under argon atmosphere. Dibromomethyl methyl ether (0.48 mL, 5.3 mmol), which was synthesized according to the method described in Gloede, J. et al. Chem. Ber., vol. 100, p. 1902 (1967), and chloroform (0.15 mL) were added to the mixture, and the mixture was stirred at room temperature for 20 hours. After the completion of the reaction, the reaction solution was separated by adding dichloromethane (15 mL) and 2 M aqueous hydrochloric acid solution (10 mL). The organic layer was washed with 2 M aqueous hydrochloric acid solution (10 mL) and saturated aqueous sodium hydrogen carbonate solution (10 mL), dried over anhydrous magnesium sulfate, and concentrated under reduced pressure. To the concentrated residue were added methanol (10 mL) and silver carbonate (I) (372 mg, 1.35 mmol) to prevent light-transmittance, and the mixture was stirred at room temperature for 14 hours. After the completion of the reaction, the solid was removed by filtration, and the filtrate was concentrated under reduced pressure. The resulting oily substance was purified by chromatography (elution 25:1 hexane-ethyl acetate) to give the object compound (106 mg, yield 48%) as white crystals.

### Example 5. Synthesis of methyl-2-deoxy-2-bromo-3,5-di-O-toluoyl-β-L-arabinofuranoside

To a suspension of zinc bromide (45 mg, 0.2 mmol) in chloroform (1 mL) were added 2,3,5-tri-O-toluoyl-α-L-arabinofuranosyl bromide (56.7 mg, 0.1 mmol) and phosphorus tribromide (0.0095 mL, 0.1 mmol), and the mixture was stirred under heating at 40°C to 20 hours. After the completion of the reaction, the reaction mixture was cooled to 0°C. Methanol (2 mL) was added to the solution, and the mixture was warmed to room temperature and stirred for 1 hour. The resulting solution was concentrated and separated by adding ethyl acetate (4 mL) and water (1 mL). The organic layer was washed with water (1 mL) and saturated brine (1 mL), dried over anhydrous magnesium sulfate, and concentrated under reduced pressure. The resulting oily substance was purified by chromatography (elution 10:1 hexane-ethyl acetate) to give the object compound (30.2 mg, yield 65%) as white crystals.

### Example 6. Synthesis of methyl-2-deoxy-2-bromo-3,5-di-O-toluoyl-β-L-arabinofuranoside

To a suspension of zinc bromide (13.5 g, 60 mmol) in chloroform (300 mL) were added phosphorus tribromide (5.7 mL, 60 mmol) and 2,3,5-tri-O-toluoyl-α-L-arabinofuranosyl bromide (17.1 g, 30 mmol), and the mixture was stirred under heating at 45°C for 14 hours. After the completion of the reaction, the reaction mixture was cooled to 0°C. Methanol (300 mL) was added to the solution, and the solution was warmed to room temperature and stirred for 1 hour. The resulting solution was concentrated, and separated by adding dichloromethane (900 mL) and water (300 mL). The organic layer was washed with water (300 mL) and saturated brine (150 mL), dried over anhydrous magnesium sulfate, and concentrated under reduced pressure. Ethanol (60 mL) was added to the resulting oily substance to precipitate crystals, and the mixture was stirred at room temperature for 14 hours. The resulting crystals were collected by filtration to give the object compound (5.6 g, yield 40%) as white crystals.

### Example 7. Synthesis of methyl-2-deoxy-2-bromo-3,5-di-O-toluoyl-f3-L-arabinofuranoside

To a suspension of zinc bromide (18 g, 80 mmol) in dichloromethane (400 mL) were added phosphorus tribromide (7.6 mL, 80 mmol), ethanol (4.67 mL, 80 mmol), and 2,3,5-tri-O-toluoyl-α-L-arabinofuranosyl bromide (22.7 g, 40 mmol), and the mixture was stirred under heating at 40°C for 14 hours. After the completion of the reaction, the reaction mixture was cooled to 0°C. Methanol (400 mL) was added to the solution, and the mixture was warmed to room temperature and stirred for 1 hour. The resulting solution was concentrated and separated by adding dichloromethane (1.2 L) and water (400 mL). The organic layer was washed with water (400 mL) and saturated brine (200 mL), dried over anhydrous magnesium sulfate, and concentrated under reduced pressure. Ethanol (80 mL) was added to the resulting oily substance to precipitate crystals, and the mixture was stirred at room temperature for 14 hours. The resulting crystals were collected by filtration to give the object compound (6.7 g, yield 36%) as white crystals.

### Example 8. Synthesis of methyl-2-deoxy-2-bromo-3,5-di-O-toluoyl-β-L-arabinofuranoside

To a solution of 2,3,5-tri-O-toluoyl-α-L-arabinofuranosyl bromide (1.0 g, 1.76 mmol) in chloroform (5 mL) was added zinc bromide (793 mg, 1.0 mmol) under argon atmosphere. To the mixture were added dibromomethyl methyl ether (2.45 mL, 14.8 mmol) and chloroform (2 mL), and the mixture was stirred under heating at 30°C for 24 hours. After the completion of the reaction, the reaction mixture was cooled and separated by adding dichloromethane (40 mL) and 2 M aqueous hydrochloric acid solution (40 mL). The organic layer was washed with 2 M aqueous hydrochloric acid solution (20 mL) and saturated aqueous sodium hydrogencarbonate solution (40 mL), dried over anhydrous sodium sulfate, and concentrated under reduced pressure. To the concentrate were added methanol (5.7 mL) and p-toluenesulfonic acid monohydrate (17 mg, 0.09 mmol), and the mixture was stirred at room temperature for 14 hours. The precipitated crystals that occurred with the progress of the reaction were collected by filtration to give the object compound (357 mg, yield 44%) as white crystals.

### Example 9. Synthesis of methyl-2-deoxy-3,5-di-O-toluoyl-β-L-ribofuranoside

To a solution of methyl-2-deoxy-2-bromo-3,5-di-O-toluoyl-β-L-arabinofuranoside (100 mg, 0.216 mmol) in acetonitrile (1 mL) were added 50% aqueous hypophosphorous acid solution (85.2 mg, 0.648 mmol), triethylamine (0.09 mL, 0.648 mmol), and 1,1'-azobis(cyclohexanecarbonitrile) (5.4 mg, 0.022 mmol), and the mixture was stirred under heating at 80°C for 20 hours. After the completion of the reaction, the reaction mixture was cooled, and separated by adding dichloromethane (10 mL) and water (30 mL). The aqueous layer was extracted again by adding dichloromethane (10 mL). The organic layers were combined, dried over anhydrous sodium sulfate, and concentrated under reduced pressure. The resulting oily substance was purified by chromatography (elution 30:1 toluene-ether) to give the object compound (63.7 mg, yield 77%) as white crystals.
¹H NMR (CDCl₃) δ 2.34 (dt, J=14.1, 5.3 Hz, 1H), 2.34 (s, 3H), 2.41 (s, 3H), 2.56 (ddd, J= 14.1, 7.2, 2.2 Hz, 1H), 3.36 (s, 3H), 4.45―4.59 (m, 3H), 5.23 (dd, J= 5.3, 2.2 Hz, 1H), 5.58―5.61 (m, 1H), 7.21 (d, J= 8.0 Hz, 2H), 7.23 (d, J= 8.0 Hz, 2H), 7.91 (d, J= 8.2 Hz, 2H), 7.93 (d, J= 8.2 Hz, 2H).

### Example 10. Synthesis of methyl-2-deoxy-3,5-di-O-toluoyl-β-L-ribofuranoside

To a mixed solution of methyl-2-deoxy-2-bromo-3,5-di-O-toluoyl-β-L-arabinofuranoside (1.0 g, 2.16 mmol) in 2-propanol (4 mL) and water (1 mL) were added triethylamine (0.3 mL, 2.16 mmol) and 10% palladium-carbon (234 mg, water content 50.5%), and the mixture was stirred under hydrogen atmosphere at 60°C for 5 hours and at 80°C for 2 hours. After the completion of the reaction, the mixture was cooled, and the palladium catalyst was filtered off. The filtrate was concentrated. Methanol (6 mL) was added to the concentrate to precipitate crystals, and the mixture was stirred at 0°C for 30 minutes. The resulting crystals were collected by filtration to give the object compound (596 mg, yield 72%) as white crystals.

### Example 11. Synthesis of methyl-2-deoxy-3,5-di-O-toluoyl-α-L-ribofuranoside

To a solution of methyl-2-deoxy-2-bromo-3,5-di-O-toluoyl-α-L-arabinofuranoside (692 mg, 1.5 mmol) in acetonitrile (6.9 mL) were added 50% aqueous hypophosphorous acid solution (0.99 mL, 7.5 mmol), triethylamine (1.05 mL, 7.5 mmol), and 1,1'-azobis(cyclohexanecarbonitrile) (37 mg, 0.15 mmol), and the mixture was stirred under heating at 80°C for 14 hours. After the completion of the reaction, the mixture was cooled and separated by adding dichloromethane (6.9 mL) and water (22.8 mL). The aqueous layer was extracted again by adding dichloromethane (6.9 mL). The organic layers were combined, dried over anhydrous magnesium sulfate, and concentrated under reduced pressure. The resulting oily substance was purified by chromatography (elution 30:1→15:1 hexane-ethyl acetate) to give the object compound (193.4 mg, yield 61%) as white crystals.
¹H NMR (CDCl₃) δ 2.19 (ddd, J= 14.5, 2.3, 1.0 Hz, 1H), 2.40 (s, 3H), 2.41 (s, 3H), 2.55 (ddd, J= 14.5, 8.2, 5.4 Hz, 1H), 3.42 (s, 3H), 4.50-4.57 (m, 2H), 4.63 (dd, J= 13.0, 4.8, Hz, 1H), 5.19 (dd, J= 5.4, 1.0 Hz, 1H), 5.41 (ddd, J= 8.2, 3.7, 2.3 Hz, 1H), 7.21 (d,1= 7.9 Hz, 2H), 7.23 (d, J= 7.9 Hz, 2H), 7.91 (d, J= 8.3 Hz, 2H), 7.93 (d, J= 8.3 Hz, 2H).

### Example 12. Synthesis of 2-deoxy-3,5-di-O-toluoyl-α-L-ribofuranosyl chloride

To a suspension of methyl-2-deoxy-3,5- di-O-toluoyl-β-L-ribofuranoside (1.0 g, 2.6 mmol) in hexane (4 mL) was added 4 M solution of hydrogen chloride in ethyl acetate (4 mL, 16 mmol) at room temperature, and the mixture was directly stirred for 1.5 hours. The precipitated crystals generated simultaneously with the progress of the reaction were collected by filtration to give the object compound (810 mg, yield 80%) as white crystals. ¹H NMR (CDCl₃) δ 2.41 (s, 3H), 2.43 (s, 3H), 2.75 (dd, J=15.1, 0.9 Hz, 1H), 2.87 (ddd, J= 15.1, 7.4, 5.1 Hz, 1H), 4.59 (dd, J= 12.1, 4.2 Hz, 1H), 4.68 (dd, J= 12.1, 3.2 Hz, 1H), 4.83―4.88 (m, 1H), 4.68 (ddd, J= 7.4, 2.9, 0.9 Hz, 1H), 6.47 (d, J= 5.1 Hz,1H), 7.23 (d, J= 8.0 Hz, 2H), 7.26 (d, J= 8.0 Hz, 2H), 7.89 (d, J= 8.2 Hz, 2H), 7.99 (d, J= 8.2 Hz, 2H).

### Example 13. Synthesis of 2-deoxy-3,5-di-O-toluoyl-α-L-ribofuranosyl chloride

To a suspension of methyl-2-deoxy-3,5-di-O-toluoyl-α-L-ribofuranoside (1.0 g, 2.6 mmol) in hexane (4 mL) was added 4 M solution of hydrogen chloride in ethyl acetate (4 mL, 16 mmol) at room temperature, and the mixture was directly stirred for 1.5 hours. The precipitated crystals that occurred with the progress of the reaction were collected by filtration to give the object compound (393.3 mg, yield 78%) as white crystals.

### Reference Example 1. 1,2-Dibromination of methyl-2,3,5-tri-O-toluoyl-L-arabinofuranoside

To a solution of methyl-2,3,5-tri-O-toluoyl-L-arabinofuranoside (51.9 mg, 0.1 mmol) in chloroform (1 mL) were added zinc bromide (45 mg, 0.2 mmol) and lithium bromide (52.1 mg, 0.6 mmol), and the mixture was stirred under heating at 40°C for 14 hours. The object compound, 2-deoxy-2-bromo-3,5-di-O-toluoyl-α-L-arabinofuranosyl bromide, was not confirmed at all, but the starting material, methyl-2,3,5-tri-O-toluoyl-L-arabinofuranoside, remained unreacted.

### Reference Example 2. Dibromination of 2,3,5-tri-O-toluoyl-α-L-arabinofuranosyl bromide

To a suspension of zinc bromide (45 mg, 0.2 mmol) in chloroform (1 mL) were added 2,3,5-tri-O-toluoyl-α-L-arabinofuranosyl bromide (56.7 mg, 0.1 mmol) and lithium bromide (52.1 mg, 0.6 mmol), and the mixture was stirred under heating at 40°C for 14 hours. The object compound, 2-deoxy-2-bromo-3,5-di-O-toluoyl-α-L-arabinofuranosyl bromide was not confirmed at all, and the starting material, 2,3,5-tri-O-toluoyl-α-L-arabinofuranosyl bromide, remained unreacted.

### Reference Example 3. Dibromination of 2,3,5-tri-O-benzoyl-α-L-arabinofuranosyl bromide

To a suspension of zinc bromide (225 mg, 1.0 mmol) in chloroform (1 mL) was added bromo-2,3,5-tri-O-benzoyl-α-L-arabinofuranoside (263 mg, 0.5 mmol), and the mixture was stirred under heating at 40°C for 14 hours. The objective compound, 2-deoxy-2-bromo-3,5-di-O-benzoyl-α-L-arabinofuranosyl bromide was not observed at all, and the starting material, 2,3,5-tri-O-toluoyl-α-L-arabinofuranosyl bromide, remained unreacted.

## Claims

1. A method of producing a 2-deoxy-L-ribofuranosyl chloride compound represented by formula (3): 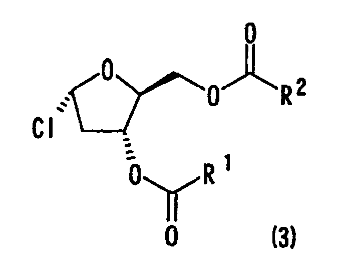 wherein R¹ and R² are each independently an aryl group optionally having one or more substituents, an alkyl group optionally having one or more substituents, or a hydrogen atom,
said method comprising:
(i) dehalogenating a 2-deoxy-2-halo-L-arabinofuranose compound represented by formula (1): 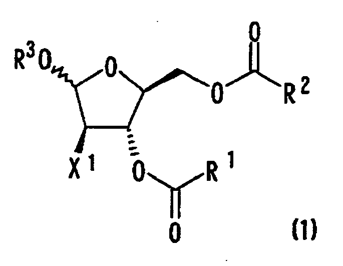 wherein R¹ and R² are as defined above, R³ is an alkyl group or an aralkyl group, and X¹ is a halogen atom, to obtain a 2-deoxy-L-ribofuranose compound represented by formula (2): 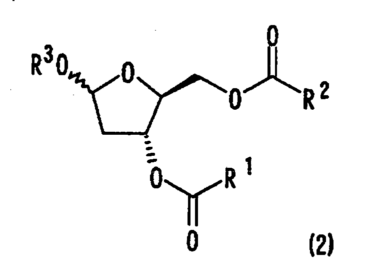 wherein R¹, R², and R³ are as defined above; and
(ii) reacting said 2-deoxy-L-ribofuranose compound represented by formula (2) with a chlorinating reagent, to obtain said compound represented by formula (3).

2. The method of claim 1, wherein said dehalogenating is carried out in the presence of at least one member selected from the group consisting of a phosphite, hypophosphorous acid, a hypophosphite, a nickel catalyst, a palladium catalyst, and mixtures thereof.

3. The method of claim 1, wherein said chlorinating reagent is at least one member selected from the group consisting of hydrogen chloride, boron chloride, magnesium chloride, aluminum chloride, trialkylsilyl chloride, phosphorus trichloride, phosphorus pentachloride, phosphorus oxychloride, thionyl chloride, sulfuryl chloride, titanium tetrachloride, zinc chloride, acetyl chloride, and mixtures thereof.

4. The method of claim 1, wherein X¹ is a bromine atom.

5. The method of claim 1, wherein both R¹ and R² are an aryl group optionally having one or more substituents.

6. A method of producing a 2-deoxy-2-halo-L-arabinofuranose compound represented by formula (1): 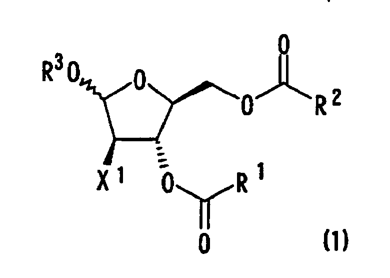 wherein R¹ and R² are each independently an aryl group optionally having one or more substituents, an alkyl group optionally having one or more substituents, or a hydrogen atom, R³ is an alkyl group or an aralkyl group, and X¹ is a halogen atom,
said method comprising:
(i) reacting:
(a) an L-arabinofuranose compound represented by formula (4): 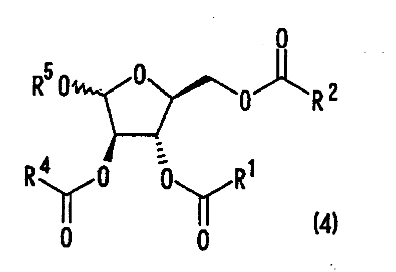 wherein R¹ and R² are as defined above, R⁴ is an aryl group optionally having one or more substituents, an alkyl group optionally having one or more substituents, or a hydrogen atom, and R⁵ is an alkyl group or an aralkyl group; or
(b) an L-arabinofuranosyl halide compound represented by formula (5): 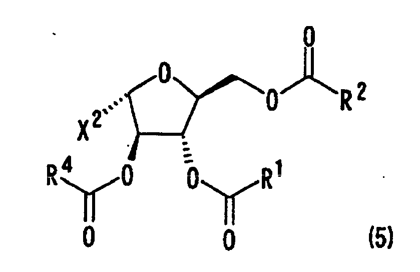 wherein R¹, R², and R⁴ are as defined above, and X² is a halogen atom,
with a halogenating reagent and an acid, to obtain a compound represented by formula (6): 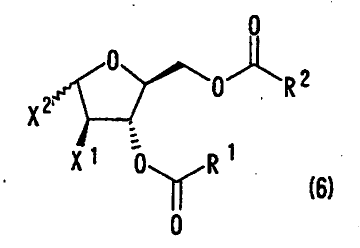 wherein X² , R¹, and R² areas defined above, and X¹ is a halogen atom; and
(ii) reacting said compound represented by formula (6) with an alcohol, to obtain said compound represented by formula (1).

7. The method of claim 6, wherein said halogenating reagent is at least one member selected from the group consisting of hydrogen bromide, phosphorus tribromide, acetyl bromide, and mixtures thereof.

8. The method of claim 6, wherein the acid is at least one member selected from the group consisting of zinc chloride, zinc bromide, aluminum bromide, and mixtures thereof.

9. A method of producing a 2-deoxy-L-ribofuranose compound represented by formula (2): 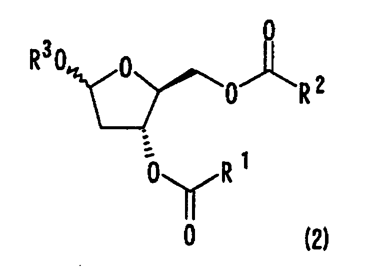 wherein R¹ and R² are each independently an aryl group optionally having one or more substituents, an alkyl group optionally having one or more substituents, or a hydrogen atom, and R³ is an alkyl group or an aralkyl group,
said method comprising:
(i) dehalogenating a 2-deoxy-2-halo-L-arabinofuranose compound represented by formula (1): 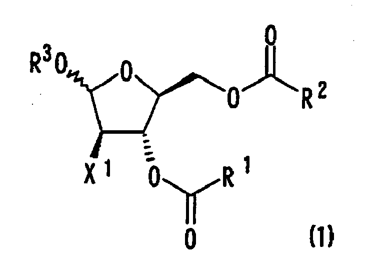
wherein R¹, R², and R³ are as defined above, and X¹ is a halogen atom.

10. The method of claim 9, wherein said dehalogenating is carried out in the presence of at least one member selected from a phosphite, hypophosphorous acid, a hypophosphite, a nickel catalyst, a palladium catalyst, and mixtures thereof.

11. The method of claim 9, wherein X¹ is a bromine atom.

12. The method of claim 9, wherein both R¹ and R² are an aryl group optionally having one or more substituents.

13. A method of producing a 2-deoxy-2-halo-L-arabinofuranose compound represented by formula (1'): 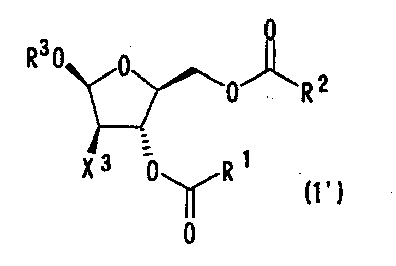 wherein R¹ and R² are each independently an aryl group optionally having one or more substituents, an alkyl group optionally having one or more substituents, or a hydrogen atom, R³ is an alkyl group or an aralkyl group and X³ is a bromine atom,
said method comprising:
(i) reacting:
(a) an L-arabinofuranose compound represented by formula (4): 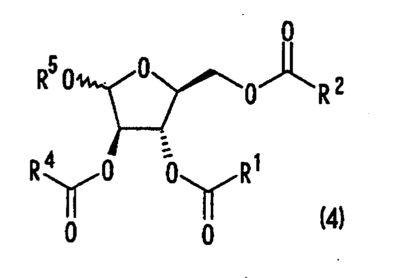 wherein R¹ and R² are as defined above, R⁴ is an aryl group optionally having one or more substituents, an alkyl group optionally having one or more substituents, or a hydrogen atom, and R⁵ is an alkyl group or an aralkyl group; or
(b) an L-arabinofuranosyl halide compound represented by formula (5): 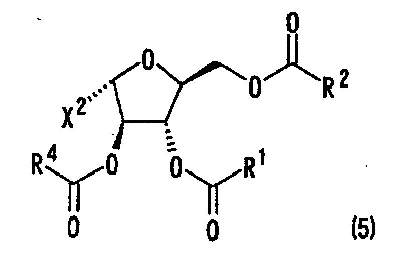 wherein R¹, R², and R⁴ are as defined above, and X² is a halogen atom,
with an acid and at least one halogenating reagent selected from the group consisting of hydrogen bromide, phosphorus tribromide, acetyl bromide, and mixtures thereof, to obtain a compound represented by formula (6): 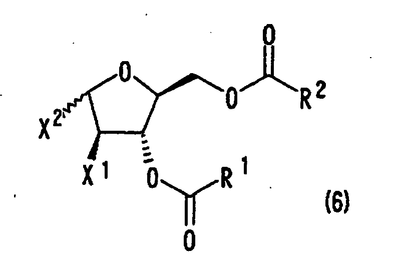 wherein X², R¹, and R² are as defined above, and X¹ is a halogen atom; and
(ii) reacting said compound represented by formula (6) with an alcohol, to obtain said compound represented by formula (1').

14. The method of claim 13, wherein the acid is at least one member selected from the group consisting of zinc chloride, zinc bromide, aluminum bromide, and mixtures thereof.

15. The method of claim 13, wherein X² is a bromine atom.

16. A 2-deoxy-2-halo-L-arabinofuranosyl halide compound represented by formula (6) 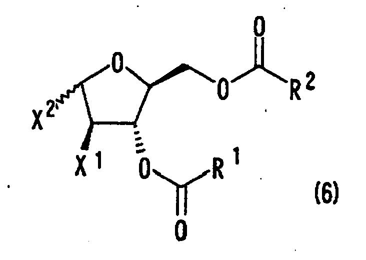 wherein R¹ and R² are each independently an aryl group optionally having one or more substituents, an alkyl group optionally having one or more substituents, or a hydrogen atom, and X¹ and X² are each independently a halogen atom.

17. The compound of claim 16, wherein both X¹ and X² are a bromine atom.

18. The compound of claim 16, wherein both R¹ and R² are an aryl group optionally having one or more substituents.

19. A 2-deoxy-2-halo-L-arabinofuranose compound represented by formula (1): 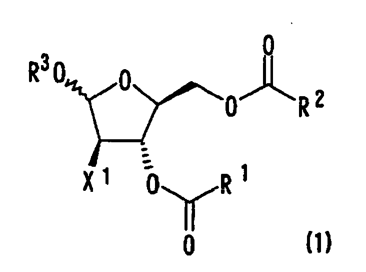 wherein R¹ and R² are each independently an aryl group optionally having one or more substituentss), an alkyl group optionally having one or more substituents, or a hydrogen atom, R³ is an alkyl group or an aralkyl group, and X¹ is a halogen atom.

20. The compound of claim 19, wherein X¹ is a bromine atom.

21. The compound of claim 19, wherein both R¹ and R² are an aryl group optionally having one or more substituents.

22. A method of producing a compound represented by formula (7): 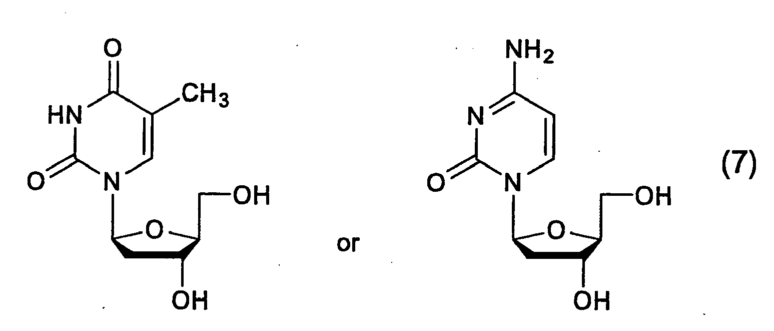 comprising converting a compound represented by formula (3): 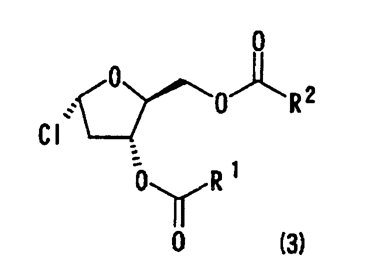 wherein R¹ and R² are each independently an aryl group optionally having one or more substituents, an alkyl group optionally having one or more substituents, or a hydrogen atom,
wherein said compound represented by formula (3) is prepared by a method comprising:
(a) dehalogenating a 2-deoxy-2-halo-L-arabinofuranose compound represented by formula (1): 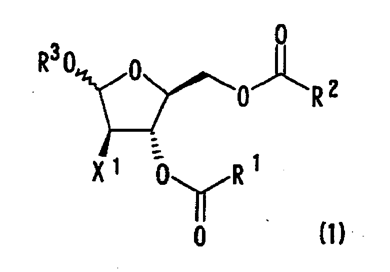 wherein R¹ and R² are as defined above, R³ is an alkyl group or an aralkyl group, and X¹ is a halogen atom, to obtain a 2-deoxy-L-ribofuranose compound represented by formula (2): 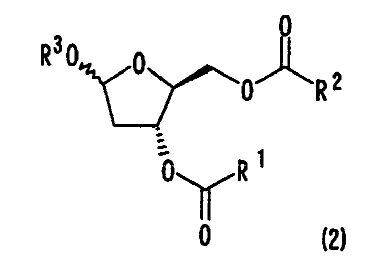 wherein R¹, R² and R³ are as defined above; and
(ii) reacting said 2-deoxy-L-ribofuranose compound represented by formula (2) with a chlorinating reagent, to obtain said compound of formula (3).

23. A method of producing a compound represented by formula (7): 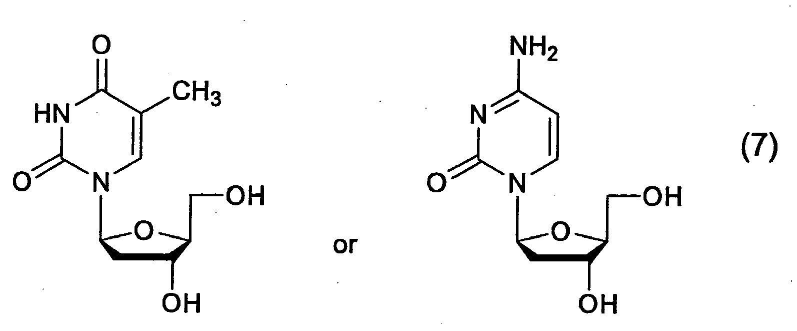 comprising converting a compound represented by formula (3): 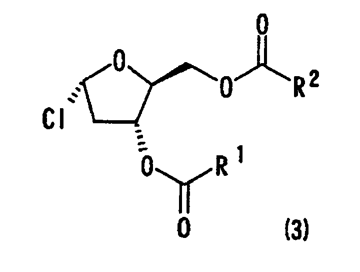 wherein R¹ and R² are each independently an aryl group optionally having one or more substituents, an alkyl group optionally having one or more substituents, or a hydrogen atom,
into said compound represented by formula (7),
the improvement being said compound represented by formula (3) is prepared by a method comprising:
(a) dehalogenating a 2-deoxy-2-halo-L-arabinofuranose compound represented by formula (1): 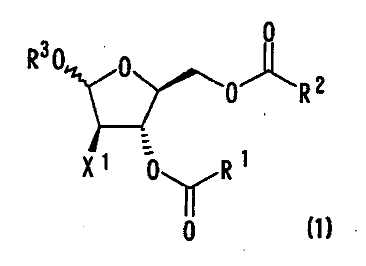 wherein R¹ and R² are as defined above, R³ is an alkyl group or an aralkyl group, and X¹ is a halogen atom, to obtain a 2-deoxy-L-ribofuranose compound represented by formula (2): 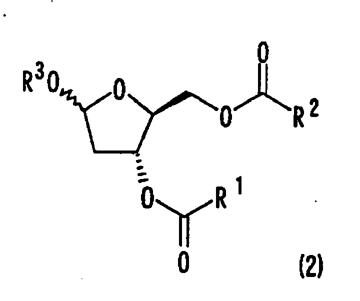 wherein R¹, R² and R³ are as defined above; and
(ii) reacting said 2-deoxy-L-ribofuranose compound represented by formula (2) with a chlorinating reagent, to obtain said compound of formula (3).
